(19) Europäisches Patentamt
European Patent Office
Office européen des brevets

(11) **EP 2 857 350 A1**

(12) **EUROPEAN PATENT APPLICATION**

(43) Date of publication:
08.04.2015 Bulletin 2015/15

(51) Int Cl.:
***B82Y 30/00*** *(2011.01)*

(21) Application number: **13306243.0**

(22) Date of filing: **10.09.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **ESPCI Innov**
**75005 Paris (FR)**

(72) Inventors:
• **Marcellain, Alba**
  **94600 Choisy-le-Roi (FR)**
• **Leibler, Ludwik**
  **75600 Paris (FR)**

(74) Representative: **Corizzi, Valérie**
**INNOVATION COMPETENCE GROUP**
**PACT- IP**
**310 bis avenue Berthelot**
**69008 Lyon (FR)**

(54) **Use of nanoparticles for gluing gels**

(57) Use of a composition of nanoparticles for gluing at least one hydrogel to at least one other article. Gel assemblies of good mechanical resistance can be obtained easily. Method for gluing at least one hydrogel to at least one other article, said method comprises: applying a composition of nanoparticles on at least one face of the hydrogel and applying the face of the hydrogel with the nanoparticles to the article.

EP 2 857 350 A1

**Description**

FIELD OF THE INVENTION

The invention relates to the use of nanoparticles, notably aqueous suspensions of nanoparticles, as a gluing agent between a hydrogel, which can be a synthetic gel or a biological tissue, and a second article, of a material which can be identical to or different from the hydrogel. It also relates to assemblies of at least one hydrogel and at least a second article, said assemblies comprising an interface of nanoparticles. The invention further relates to gluing kits based on nanoparticle, notably on nanoparticles suspensions. Using a composition of nanoparticles, notably a suspension of nanoparticles for gluing a gel to another article finds applications in numerous technical fields, like microfluidics, laboratory equipment, actuation, surgery, tissue engineering, drug delivery, agrochemical industry.

BACKGROUND OF THE INVENTION

Synthetic gels find numerous applications, however, when using hydrogels, one difficulty lies in creating a bonding between a gel and another article, of an identical or different material. First, gluing a gel to another article in itself is difficult, then creating a bond which is resistant to the conditions of use of the assembly is another difficulty. Hydrogels are fragile materials, but assemblies including a gel are often characterized by a weak bonding of the gel to other parts of the assembly, whatever their nature.

Adhesives are generally made of polymers (Kendall, K. Molecular Adhesion and Its Applications, Plenum Publishing Corporation, 2001). Polymers, in contrast to other materials, are able to ensure good contact by filling surface asperities and retard fracture of adhesive joint by dissipating energy under stress (Lake, G. J. & Thomas, A. G. Proceedings of the Royal Society A: Mathematical, Physical and Engineering Sciences 300, 108-119 (1967) ; De Gennes, P. G., Langmuir 12, 4497-4500 (1996)). Paradoxically, using polymers to assemble polymer gels is challenging and forming an adhesive junction requires chemical reactions, heating, changing pH, using UV irradiation or applying electric field (Sahlin, J. J. & Peppas, N. A., Journal of Biomaterials Science, Polymer Edition 8, 421-436 (1997) ; Tamagawa, H. & Takahashi, Y., Materials Chemistry and Physics 107, 164-170 (2008) ; Saito, J. et al., Polymer Chemistry 2, 575 (2011) ; Techawanitchai, P. et al., Soft Matter 8, 2844 (2012)).

Most often, when brought into contact and pressed together two pieces of an elastic gel do not stick and gel-gel friction is very low (Gong, J. P., Soft Matter 2, 544 (2006)). Incorporating supramolecular or covalent reversible bonds in polymers enables one to produce gels that are self-adhesive (Pezron, E., Ricard, A. & Leibler, L., J. Polym. Sci. B Polym. Phys. 28, 2445-2461 (1990) ; Bosman, A. W., Sijbesma, R. P. & Meijer, E. W., Materials Today, 34-39 (2004) ; Reutenauer, P., Buhler, E., Boul, P. J., Candau, S. J. & Lehn, J.-M., Chemistry 15, 1893-1900 (2009) ; Nicolaÿ, R., Kamada, J., Van Wassen, A. & Matyjaszewski, K., Macromolecules 43, 4355-4361 (2010) ; Imato, K. et al., Angew. Chem. Int. Ed. 51, 1138-1142 (2011)). However, manipulating self-adhesive gels is not always practical. A solution could be to use supramolecular networks with surfaces that are not self-adhesive, but which are able to self-heal when cut into pieces (Cordier, P., Toumilhac, F., Soulié-Ziakovic, C. & Leibler, L., Nature 451, 977-980 (2008) ; Maes, F. et al., Soft Matter 8, 1681-1687 (2012)). Nanocomposites comprising a hydrogel wherein nanoparticles are dispersed have been disclosed by different authors. Pressure sensitive adhesive based on hydrogel nanocomposites have been suggested for skin contact applications (Baït N. et al., Soft Matter 7, 2025-2032 (2011)). Notably, dispersing clay particles in polymer solutions or networks yields gels that combine high elasticity and toughness with self-healing capabilities (Wang, Q. et al., Nature 463, 339-343 (2010) ; Haraguchi, K., Uyama, K. & Tanimoto, H. Macromol. Rapid Commun. 32, 1253-1258 (2011)). In this design clay particles play a role of reversible cross-links (Carlsson, L., Rose, S., Hourdet, D. & Marcellan, A., Soft Matter 6, 3619-3631 (2010); Gaharwar, A. K., Rivera, C. P., Wu, C.-J. & Schmidt, G., Acta Biomaterialia 7, 4139-4148 (2011)). Still, any particular gel cannot answer all demands and it remains desirable to find a practical and universal means to glue together any biological or synthetic gel or biological tissues.

Further these prior art solutions for gel adhesion are not deprived of inconvenient. Particularly, it has been noted that the dispersion of nanoparticles in a gel material modifies the intrinsic properties of the gel, notably, it increases its stiffness.

In microfluidics, hydrogels are used as valves: When submitted to appropriate stimuli, they swell in a reversible manner and thus can be used to block and open microchannels. However, swelling and unswelling creates a strain at the interface between the gel and the carrier to which it is glued. Repeated use degrades the quality of the valve efficiency.

In actuation, it is usual to have two gels of different nature adhere and through stimulation of one gel, provoke deformation of the assembly. Here again, failures appear at the interface when submitted to deformation.

Soft biological tissues although incomparably more complex, mechanically and osmotically, resemble gels in many respects. In the medical and chirurgical field, for many applications, it is desirable to have two biological tissues adhere to one another, or to have a synthetic gel material (patch, dressing, implant, prosthesis, amniocentesis) adhere to a biological tissue. Adhesion should be sufficient until natural tissue repair occurs through cell colonization or to allow suturing. Adhesion should also be obtained without forming scars. Surgical adhesives known to date are numerous and of varied origin, but not deprived of inconvenient (Duarte A.P. et al., Progress in Polymer Science, 37, 1031-1050 (2012)).

In the field of food chemistry, the question of assembling compositions of matter is also important. The assembly must be comestible and gluing of different materials should be effective but discreet. No satisfying solution of general application for gluing edible gels exists today.

It is known to use gluing compositions comprising polymers for gluing synthetic gels. It is also known to use synthetic or natural polymer materials as surgical glue. Some adhesive compositions comprising known adhesive polymers, said compositions may comprise nanoparticles as active principles or as filler in order to increase the fracture resistance of the assembly without leading to an increase in the viscosity of the adhesive (Johnsen, B. B., A. J. Kinloch, et al. (2007). "Toughening mechanisms of nanoparticle-modified epoxy polymers." Polymer 48(2): 530-541. ; Kinloch, A. J. (1987). Adhesion and Adhesives: Science and Technology, Springer. (Kendall, K. Molecular Adhesion and Its Applications, Plenum Publishing Corporation, 2001). However these compositions were not used for gluing gels and, in these prior art compositions, nanoparticles were present as a minor component as compared to the adhesive agent, and it has never been mentioned or suggested that nanoparticles themselves could act as adhesive agent between two materials, among which, one at least, is a hydrogel.

For example, US2012/0220911 discloses glass micro particles based on a glass former and at least one trace element (Ag, Fe, Cu, F, etc...) for their use in surgical glues. However, such micro particles are formulated with an adhesive, and their function is to promote wound healing.

There remained the need of a gluing solution for gels which would be applicable to gels of synthetic nature and of biological origin, that would be easy to use, cheap, which could provide an adhesion of satisfying resistance (at least superior to the limit of resistance of the gel itself), and of esthetic appearance.

SUMMARY OF THE INVENTION

The object of the present invention is to alleviate at least partly the above mentioned drawbacks.

The invention is directed to the use of nanoparticles as gluing agent between at least one hydrogel and at least one other article.

More particularly, the invention aims to provide a method of gluing at least one hydrogel to at least one other article, of an identical or different material, in a simple, efficient and cheap manner. The invention aims to provide a method which is applicable to a wide variety of hydrogels, of synthetic or natural origin. The method according to the invention aims to provide an assembly which is resistant to external stress, notably, the adhesion of the hydrogel to the other article is superior or equal to the limit of resistance of the gel itself. Gluing by nanoparticles is surprising since powdering surfaces with micron-sized particles such as talc provides a standard means of preventing self-adhesion.

This object is achieved with a composition of nanoparticles which is used for gluing at least one hydrogel to at least one other article, wherein the nanoparticles represent from 10 to 100% by weight of the dry matter of the composition.

Additionally, the invention is directed to a method for gluing at least one hydrogel to at least one other article, wherein said method comprises:

    a- applying a composition of nanoparticles on at least one face of the hydrogel

    b- applying the face of the hydrogel bearing the nanoparticles to the article,

and wherein in case the at least one hydrogel or the at least one other article is a biological tissue, it is an isolated tissue or a cultured tissue.

The invention is also directed to an assembly of at least one hydrogel and at least one other article, wherein the interface between the at least one hydrogel and the at least one other article is a layer of nanoparticles, and wherein in case the at least one hydrogel or the at least one other article is a biological tissue, it is an isolated tissue or a cultured tissue.

The invention is also directed to a kit for gluing a hydrogel to an article, wherein said kit comprises at least a hydrogel and at least a composition of nanoparticles.

The invention is also directed to a surgical kit, wherein it comprises at least one composition of nanoparticles and at least one article chosen from: a suturing needle, a suturing strip, suturing staples, a prosthesis.

Preferred embodiments comprises one or more of the following features:

    A composition of nanoparticles wherein it is an aqueous suspension of nanoparticles.

A composition of nanoparticles, wherein the nanoparticles are selected from: clays, silicates, alumina, silica, kaolin, grafted carbon nanotubes, grafted cellulose nanocrystals, hydroxyapatite, magnetic nanoparticles.

A composition of nanoparticles, wherein the nanoparticles have an average particle size from 1 nm to 1000 nm, preferably from 2 nm to 500 nm even more preferably from 5 nm to 300 nm.

A composition of nanoparticles, wherein the hydrogel is of a material selected from: PDMA, poly-NIPAM, a synthetic or extracted gel based on proteins, a synthetic or extracted gel based on polysaccharides.

A composition of nanoparticles, wherein the hydrogel is a biological tissue.

A composition of nanoparticles, wherein the other article is of a material selected from: a hydrogel, a glass, a polymer, a biological tissue.

The invention is further directed to a method for gluing a hydrogel to another article, wherein step b is achieved underwater.

The invention is further directed to an assembly of a hydrogel and another article, wherein the concentration of nanoparticles at the interface is from 0.1 mg/m$^2$ to 10 g/m$^2$.

An assembly of a hydrogel and another article, wherein the adhesion of the hydrogel to the other article is superior to the hydrogel's resistance and is superior to the other article's resistance.

An assembly of a hydrogel and another article, wherein it is selected from: a microfluidic equipment, a biochip, a gel permeation equipment, an actuation gel assembly, an edible gel assembly, a cosmetic or pharmaceutical patch or dressing, a biosensor, a medical electrode, a contact lens, an implant, a prosthesis, a surgical strip.

Further features and advantages of the invention will appear from the following description of embodiments of the invention, given as non-limiting examples, with reference to the accompanying drawings listed hereunder.

## BRIEF DESCRIPTION OF THE DRAWINGS

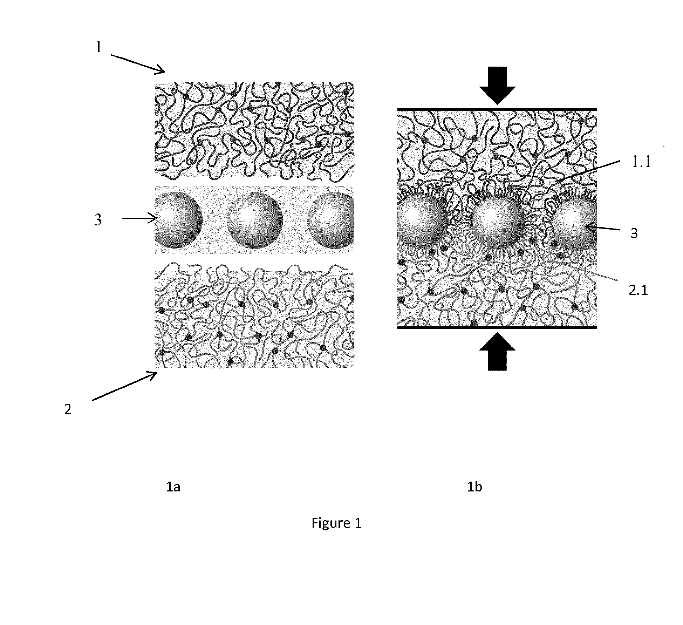

Fig. 1 shows a schematic representation of the formation of an assembly of two hydrogels with a layer of nanoparticles between the two gels.

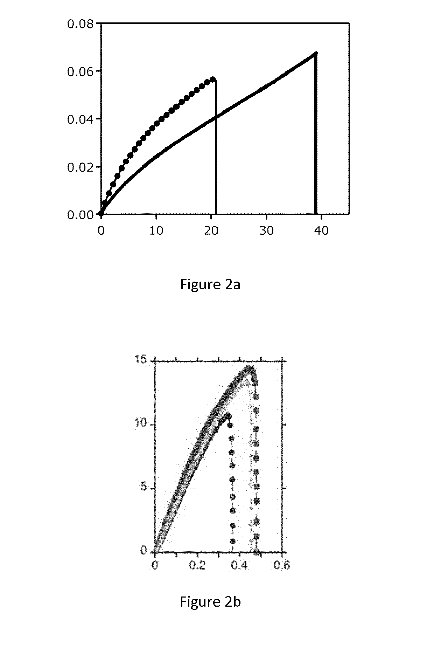

Fig. 2 shows graphics illustrating lap-shear adhesion tests.

Figure 2a includes a comparison of force (ordinate, in N) - displacement (abscissa, in mm) curves for PDMA S0.1 ribbon (continuous line) and for the lap-joint glued by spreading of 15 $\mu$L of TM-50 silica solution (doted line). Displacement is measured by optical extensometer from two markers, initially spaced by 20 mm and centered on the joint. The ribbons were $w$ = 5mm wide and $h$=2mm thick. The overlap length is $l$=10mm The PDMA S0.1/S0.1 assemblies break outside the joint.

Figure 2b illustrates a lap-shear test yielding quantitative measurement of adhesion energy of glued PDMA S01 gels. The experiment was repeated three times. Figure 2b is a graph representing normalized force (ordinate in N/m) - strain (abscissa) curves for PDMA S0.1/S0.1 lap-joint with the overlap length of $l$=5 mm glued with 6 $\mu$L of TM-50 silica solution. Adhesive failure by interfacial peeling was observed. All gel ribbons were cut from the same gel plate and the tensile modulus was measured to be $E \approx 8.1 \pm 1.0$. In these tests $w$=5mm and $h$=2mm.

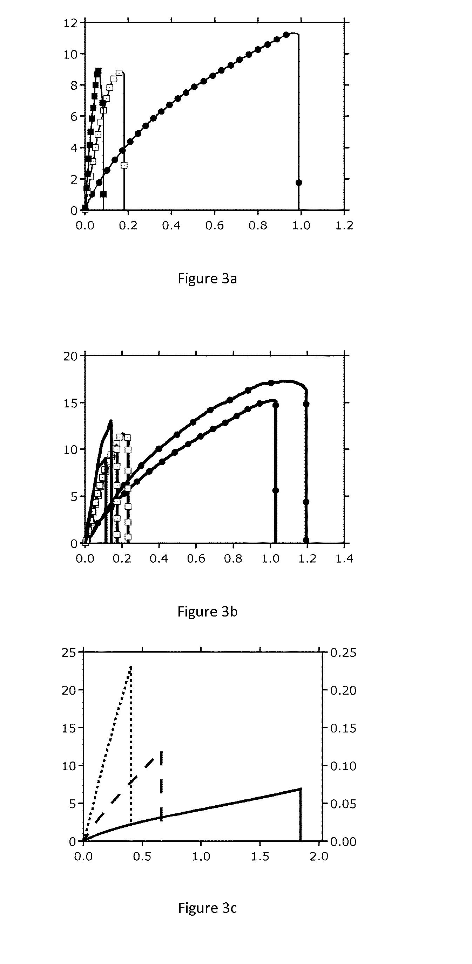

Fig. 3 shows graphics illustrating lap-shear adhesion tests of PDMA gels with different crosslinking densities (0.1mol%, 0.5 mol.% and 1.5 mol.%) glued by TM-50 particles.

Figure 3a is a graph representing normalized force (ordinate, in N.m$^{-1}$) - strain (abscissa) curves for lap joint with overlap length of 10 mm: S0.1/S0.1 (● line), S0.5/S0.5 (D line) and S1.5/S1.5 (■ line) PDMA gel assemblies. Fracture occured outside the joint for all assemblies ($w$=5mm and $h$=2mm)

Figure 3b is a graph representing normalized force (ordinate, in N.m$^{-1}$) - strain (abscissa) curves for lap joint with overlap length of 5 mm: S0.1/S0.1 (● line), S0.5/S0.5 (D line) and S1.0/S1.0 (solid line) PDMA gel assemblies. Adhesive interfacial fracture occurred outside the joint for assemblies S0.5/S0.5 and S1.0/S1.0 whereas bulk fracture occurred for S01/S01 assembly ($w$=5mm and $h$=2mm).

Figure 3c is a graph representing stress on the left axis (ordinate in kPa) and Force on the right axis (ordinate in N) as a function of strain (abscissa) and illustrates the effect of cross-linking density on the gel tensile behaviour: 50.1 (continuous line), S0.5 (long dashed line) and S1.5 (short dashed line). Strain is calculated from optical extensometer and stress is defined as engineering stress.

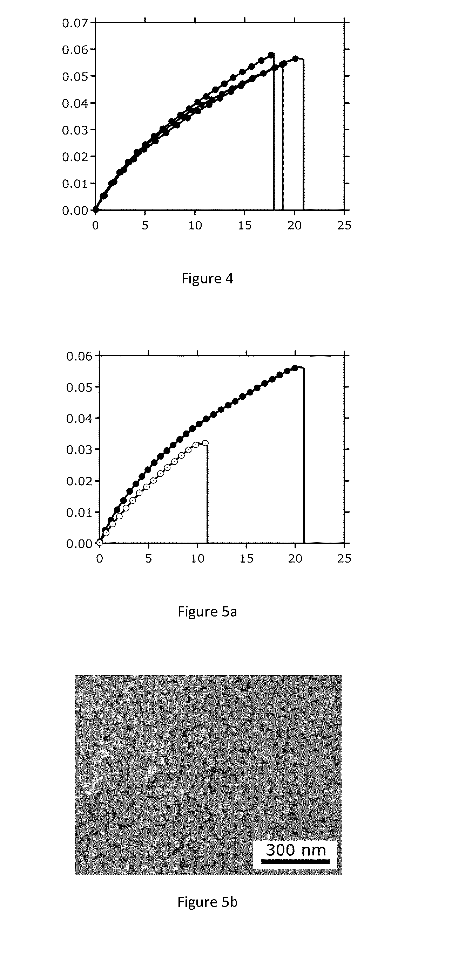

Fig. 4 shows force (ordinate, in N) - displacement (abscissa, in mm) curves for PDMA S0.1/S0.1 lap-joint with the overlap length of 10 mm, glued with TM-50 silica solution. All gel ribbons had w=5 mm and h=2mm and were cut from the same gel plate. Bulk failure outside the joint was systematically observed.

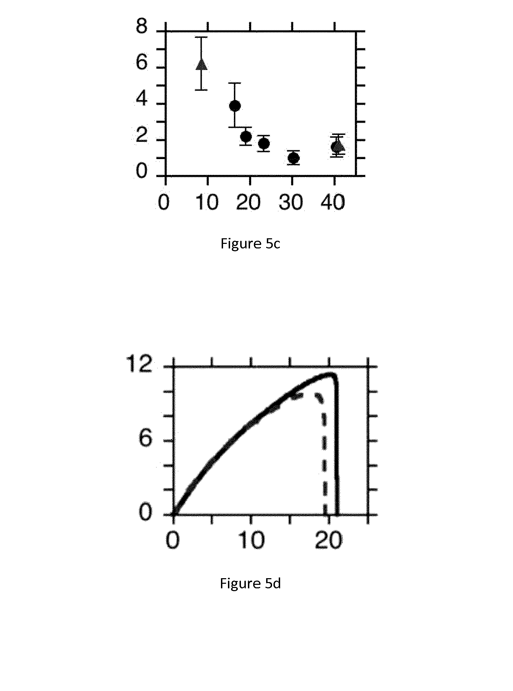

Fig. 5 illustrates water resistant adhesion and energy dissipation. Figure 5a is a graphic representing lap-shear test for PDMA S0.1/S0.1 assembly glued with TM-50 solution at the preparation state, $Q_0$ (● line) and after swelling in water for 3 days, $Q_e$ (○ line) and attaining maximum equilibrium swelling Qe. Force in Newton (ordinate, N) is represented as a function of displacement (abscissa, mm).

Figure 5b is a scanning electron micrograph showing the presence of adsorbed silica layer, which persisted after multiple washing and soaking of the 50.1 gel surface in water for several days.

Figure 5c is a graph representing adhesion energy (ordinate, in J.m$^{-2}$) - swelling degree (abscissa) curves of joints made of PDMA 50.1 hydrogels swollen prior to being glued with AL-30 silica solutions to various degrees of swelling Q (●) and adhesion energy of joints made of 50.1 hydrogels glued with TM-50 silica solutions at as-synthesized swelling degree ($Q_0 \approx 8.5$) and after being immersed in water and swollen to reach the maximum, equilibrium swelling degree, $Q_e \approx 41$ (▲).

Figure 5d is a graph representing lap-shear test for PDMA S0.1/S0.1 glued by spreading 6$\mu$L droplet of HS-40 silica solution (continuous line). After adhesive interfacial failure by peeling the joint was repaired by bringing ribbons back to contact and pressing with fingers for a dozen of seconds. The joint recovered its strength (dashed line). The lap joints were 5 mm wide, 2 mm thick and the overlap length was 5 mm.

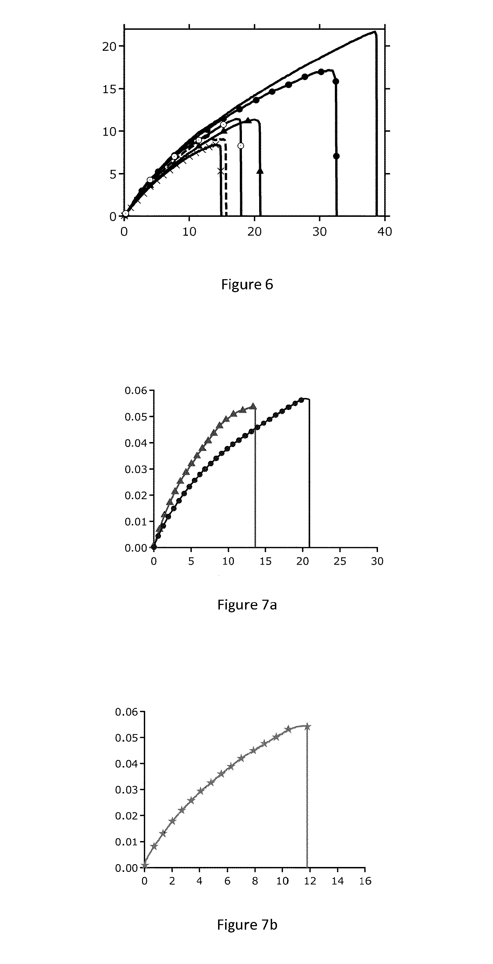

Fig. 6 illustrates lap-shear test for PDMA S01 gels glued by various particle solutions. In figure 6, force (ordinate, N/m) is represented as a function of displacement (abscissa, mm). In order of increasing deformation at break: adhesive failure by interfacial peeling occured for CNT-Thy (× line), CNC1 (dotted line), SM-30 (○ line), and HS-40 (▲ line), and the fracture outside the junction occurred for TM-50 (●) and AL-30 (continuous line). Lap joint dimensions were *l*=5mm *w*=5mm and *h*=2mm. 6$\mu$L of solutions was spread to make the junction.

Figure 7 illustrates tensile test of lap-joints made of gels of different stiffness or chemical nature. Figure 7a: Lap-shear force (ordinate in N) - displacement (abscissa, in mm) curve for an assembly made of soft PDMA 50.1 and rigid PDMA S1.5 (A) gels. For comparison the results obtained under identical conditions for the symmetric PDMA S0.1/S0.1 assembly are plotted (●). Lap joint dimensions were *l*=10mm *w*=5mm and *h*=2mm. 15$\mu$L of TM-50 solution was spread to make the junction. Figure 7b, Lap-shear force (ordinate in N) - displacement (abscissa, in mm) curve for gelatin and 50.1 PDMA gel assembly glued with TM-50 silica solution (★). The 50.1 and gelatin gel had different rigidity.

Silica nanoparticles enable one to glue the gels of different chemical natures and of different mechanical properties. For both PDMA S0.1/PDMA S0.5 and PDMA S0.1/gelatin assemblies, the failure occurred outside the lap joint and cracks propagated in tension mode.

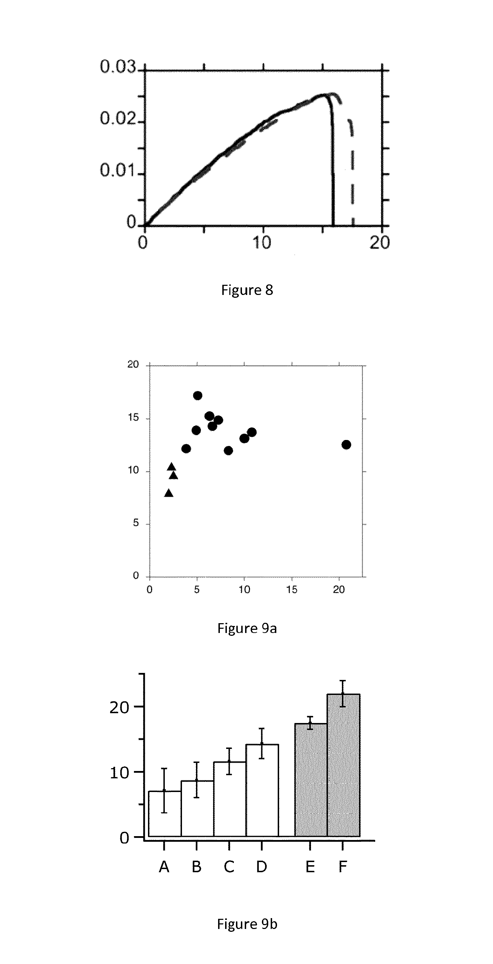

Figure 8 illustrates how junctions glued by cellulose nanocrystals CNC1 are self-repairable and repositionable. Figure 8 is a graph showing lap-shear force (ordinate in N) - displacement (abscissa, in mm) curve for PDMA S0.1/S0.1 glued by spreading 6$\mu$L droplet of CNC1 solution (continuous line). After adhesive interfacial failure by peeling, the joint was repaired by putting ribbons back to contact and pressing with fingers for a dozen of seconds. The joint recovered its strength (dashed line). There is no need to re-apply glue. The lap joints were 5 mm wide, 2 mm thick and the overlap length was 5 mm.

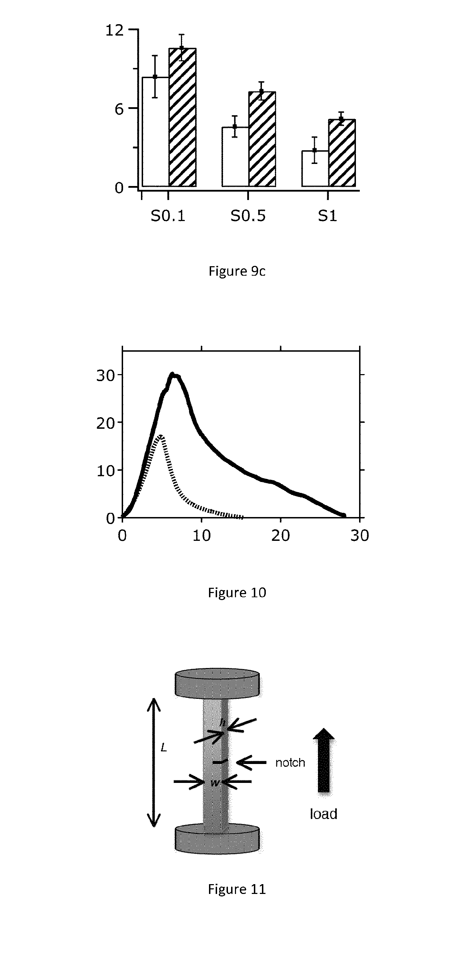

Fig. 9 illustrates lap-shear adhesion tests. Figure 9a is a graph representing the normalized failure force (ordinate, in N.m$^{-1}$) measured in lap-shear adhesion test for lap joints of various overlap length *l* (abscissa, in mm) made of 50.1 gels ribbons glued by TM-50 silica solutions. Circles correspond to failure by fracture outside the joint and triangles to adhesive failure by peeling at the interface. Ribbons were 5 mm wide and 2 mm thick. Figure 9b: is a graph representing normalized failure force (ordinate in N/m) measured in lap-shear adhesion test for lap joints glued using solutions of various particles: carbon nanotubes CNT-Thy (A), cellulose nanocrystals CNC1 (B), and silica of various sizes SM-30 (C), HS-40 (D), TM-50 (E), and AL-30 (F). For particle solutions E and F fracture occurred outside the joint, for A, B, C, and D adhesive failure by peeling was observed. The lap joints were 5 mm wide, 2 mm thick and the overlap length was 5 mm. Figure 9c is a graph representing adhesion energy $G_{adh}$ (unfilled bars, ordinate in J/m$^2$) measured in lapshear test and fracture energy $G_c$ (striped bars, ordinate in J/m$^2$) measured in single edge notch tensile test of PDMA gels of various cross-linking densities 0.1mol% (S0.1), 0.5 mol% (S0.5), and 1 mol% (S1.0). Lap joints were glued with TM-50 silica solutions.

Figure 10 is a graph representing normalized force (ordinate in N/m) - displacement (abscissa, in mm) curves for lap-joints made of ribbons cut from calf liver and glued by spreading 60$\mu$L of TM-50 silica solution and pressing ribbons with a finger for 30 seconds. The ribbons were cut with a scalpel blade and no treatment was applied to liver surfaces before gluing. The moduli of two livers are respectively 15.0±1.7 kPa and 12±1.5 kPa. The ribbons were 10 mm wide, 3 mm thick and the overlap length was 20 mm. Results for two livers are presented.

Figure 11 is a drawing which schematically illustrates the single notch fracture test method.

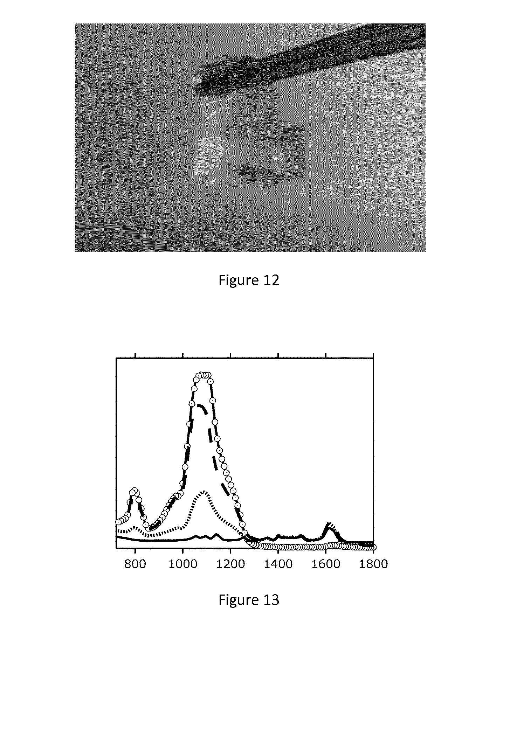

Figure 12 is a photograph of a PDMA S0.1/S1.5 assembly after immersion in water. Glued at their preparation state by TM-50 solution, both PDMA 50.1 and PDMA S1.5 gels had initially the same size (diameter of about 10 mm). Picture shows gels after 5 hours of swelling in deionised water. Highly cross-linked PDMA S1.5 gel (top piece) is less swollen than PDMA 50.1 gel (bottom piece). Gel S01 shows a fivefold over-swelling when immersed in water, whereas the more tightly cross-linked gel S1.5 over-swells by a factor of 1.7 only. As a result, interfacial stresses induced by heterogeneous over-swelling exceed considerably shear stresses applied in mechanical lap-shear tests. Hence, for S0.1/S1.5 assemblies, interfacial failure was observed after more than 5 hours of immersion and over-swelling in water. Still adhesion joint held for quite a long time and the de-bonding was slow.

Fig. 13 is a FTIR-ATR spectra showing the remaining adsorbed silica particles onto a S0.1 gel surface at $Q_0$ (long dashed line) and $Q_e$ (short dashed line) after soaking and washing in water. Dried silica suspension (○ line) and 50.1 dried gel (solid line) are presented as guideline.

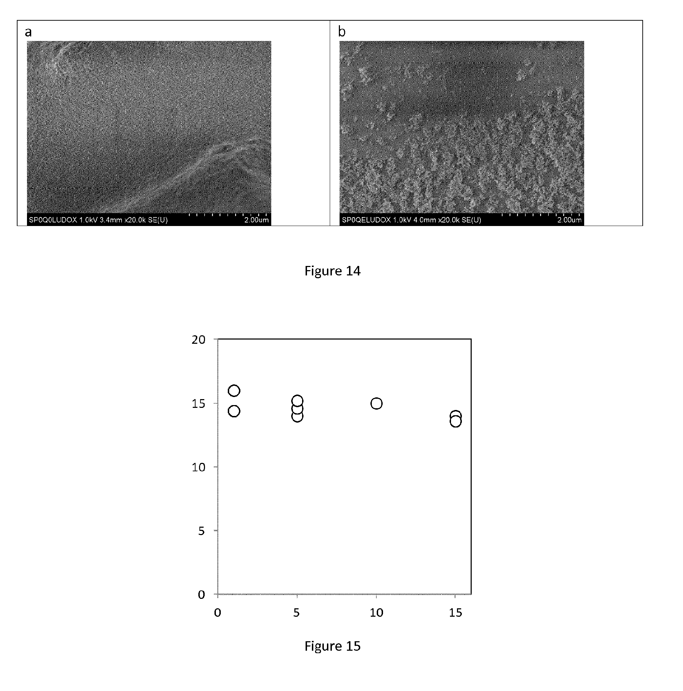

Fig. 14: Scanning Electron Microscopy (SEM) of adsorbed silica particles onto a 50.1 gel surface at $Q_0$ (figure 14a) and $Q_e$ (figure 14b), after water soaking and washing.

Fig.15 is a graph representing normalized force (ordinate in N/m) - volume of silica suspension volume (abscissa, in $\mu$l) results for lap-joints made of PDMA 50.1 and glued by spreading TM-50 silica solution and applying to ribbons a pressure of 10kPa for 30 seconds. The ribbons were 5 mm wide, 2 mm thick and the overlap length was 15 mm.

DETAILED DESCRIPTION OF THE INVENTION

The invention is directed to the use of a composition of nanoparticles, notably an aqueous suspension of nanoparticles for gluing at least one hydrogel to at least one other article.

According to the present invention, by "gluing", it is also meant "adhering", "bonding", "adhesive bonding" or "fixing by an adhesive".

Preferably, the adhesion is measured by a lap-shear test according to the protocol disclosed in the experimental part. Preferably, the adhesion of the hydrogel to the other article is superior to the hydrogel's resistance and to the other article's resistance. It means that, in this test, failure occurs outside the lap joint. However good adhesion but interfacial failure or peeling can occur in some cases.

- Composition of nanoparticles :

The term "nanoparticles" means particles from 1 nm to 1000 nm, preferably from 2 to 500 nm and even more preferably from 5 to 300 nm in size. For most nanoparticles, the size of the nanoparticles is the distance between the two most distant points in the nanoparticle. For anisotropic nanoparticles, such as tubes whiskers or cylinders, the size of the diameter is the diameter of the smallest cylinder in which the nanoparticle is inscribed. Nanoparticle size can be determined by different methods such as Dynamic Light Scattering (DLS), Small Angle X-ray Scattering (SAXS), Scanning Mobility Particle Sizer (SMPS), Scanning Electron Microscopy (SEM), Transmission Electron Microscopy (TEM) (Orts-Gil, G., K. Natte, et al. (2011), Journal of Nanoparticle Research 13(4): 1593-1604; Alexandridis, P. and B. Lindman (2000), Amphiphilic Block Copolymers: Self-Assembly and Applications, Elsevier Science; Hunter, R. J. and L. R. White (1987). Foundations of colloid science, Clarendon Press.).

Preferably, nanoparticles are selected among solid nanoparticles.

Nanoparticles can be inorganic, organic or mixed, and be coated or grafted.

The compositions, preferably the aqueous suspensions of nanoparticles which can be used according to the invention can comprise nanoparticles of different chemical nature, of different sizes, and/or of different shapes.

The nanoparticles can be in the form of a sphere, needle, flake, platelet, tube, fiber, cube, prism, whiskers or have an irregular shape.

Nanoparticles include without limitation the nanofibrils, nanochips, nanolatexes, nanotubes, expandable nanoparticles.

As indicated above, the nanoparticles can be inorganic, organic or mixed.

Among the mineral nanoparticles, one can mention metal oxides, clays, silicates, alumina, silica, kaolin, hydroxyapatite, calcium carbonate.

Mineral particles may include, but are not limited to, metal particles. Metal particles encompass particles formed exclusively with metals chosen among alkaline earth metal, transitional metal, rare earth metal, and alloys thereof. In some embodiments, the metal may be aluminum, copper, cadmium, selenium, silver, gold, indium, iron, platinum, nickel, molybdenum, silicon, titanium, tungsten, antimony, palladium, zinc, tin, and alloys thereof. These metal particles may be metal organo modified nanoparticles having chemical entities grafted to their surface or having a self-assembled monolayer of compounds, such as organosulfur compounds, on their surface.

In some embodiments, particles may be particles of metal oxides, such as iron oxides (FeO, Fe2O3, Fe3O4) cerium oxide (CeO), alumina (A1203), zirconium oxide (ZrO2), titanium oxide (TiO2), titanates (BaTiO3, Ba0.5Sr0.5TiO3, SrTiO3), indium oxide (In2O3), tin oxide (SnO2), antimony oxide (Sb2O3), magnesium oxide (MgO), calcium oxide (CaO), manganese oxides (Mn3O4, MnO2), molybdenum oxide (MoO3), silica (SiO2), zinc oxide (ZnO), yttrium oxide (Y2O3), bismuth oxychloride.

Particles may be metal carbides, nitrides, borides, sulphides and hydroxides.

They can also be organo-metallic nanoparticles: they are metal or metal oxide, carbides, nitrides, borides, sulphides and hydroxides nanoparticles, coated or grafted by an organic material.

Nanoparticles can be selected among metal inorganic salts: Inorganic salts include barium sulfate, calcium carbonate, calcium sulfate, calcium phosphate, magnesium hydrogen carbonate.

Nanoparticles can be selected among metal soaps derived from organic carboxylic acids having from 8 to 22 carbon atoms, preferably from 12 to 18 carbon atoms, for instance zinc stearate, magnesium or lithium stearate, zinc laurate, magnesium myristate.

Nanocomposite particles are included in the scope of the invention like for example core/shell metal/silica nanoparticles.

The particles can also be organic.

When the particle is organic, it is usually an organic polymer.Organic polymers encompass, but are not limited to, polystyrene, poly(vinyl acetate), poly(methylstyrene), poly(acrylamide), poly(acrylonitrile), poly(vinyl chloride), copolymers of styrene and C1-C4alkyl (meth)acrylate, copolymers of styrene and acrylamide, copolymers of styrene and acrylonitrile, copolymers of styrene and vinyl acetate, copolymers of acrylamide and C1-C4 alkyl (meth)acrylates, copolymers from acrylonitrile and C1-C4 alkyl (meth)acrylate, copolymers of acrylonitrile and acrylamide, terpolymers from styrene, acrylonitrile and acrylamide, poly(methyl methacrylate), poly(ethyl methacrylate), copolymers styrene/butadiene, styrene/acrylic acid, styrene/vinylpyrrolidone and butadiene/acrylonitrile.

For instance, organic nanoparticles include, but are not limited to, nylon (for example marketed by ATOCHEM), polyethylene powders (for example marketed by PLAST LABOR), poly-2-alanine powders, polyfluorinated powders such as polytetrafluoroethylene (for example marketed by DUPONT DE NEMOURS), acrylic copolymer powders (for example marketed by DOW CHEMICA), polystyrene powders (for example marketed by PRESPERESE), polyester powders, expanded microspheres in thermoplastic material (for example marketed by EXPANCEL), microballs of silicon resins (for example marketed by TOSHIBA), synthetic hydrophilic polymer powders such as polyacrylates (for example marketed by MATSUMOTO), acrylic polyamides (for example marketed by ORIS), insoluble polyurethanes (for example marketed by TOSHNU), porous microspheres of cellulose, micro- or nanoparticles of PTFE (polytetrafluoroethylene).

Preferably, the nanoparticles are inorganic. Even more preferably, they are selected from: clays, silicates, alumina, silica, kaolin, grafted carbon nanotubes, grafted cellulose nanocrystals, hydroxyapatite, magnetic nanoparticles like iron oxides, calcium carbonate.

According to a favorite variant, at least part of the nanoparticles are silica nanoparticles. Advantageously, the aqueous suspension is an aqueous suspension of colloidal silica. Nanoparticles which are used in the invention are selected as a function of the hydrogel's nature. The nanoparticles should be capable of adsorption at the hydrogel's surface. The selection of the appropriate nanoparticles suspension can be achieved by testing the nanoparticles affinity (adsorption) with the hydrogel. Testing methods are disclosed in a detailed manner in the experimental part.

Nanoparticles can additionally be selected as a function of the other article's nature. The nanoparticles should preferably be capable of adsorption at the other article's surface. The selection of the appropriate nanoparticles suspension can be achieved by testing the nanoparticles affinity (adsorption) with the other article by using the same methods.

Briefly, a first method rests on Fourier transform infrared spectroscopy coupled with ATR. Attenuated total reflectance (ATR) is a sampling technique used in conjunction with infrared spectroscopy which enables sample surfaces to be examined. The detection and the quantification of adsorbed nanoparticles layer onto the gel surface can be achieved. The proposed method consists in immersing the gel sample into the nanoparticle solution or depositing a droplet of nanoparticle solution on the gel surface, then the gel sample is soaked and washed in a large volume of water during several days. Samples can be dried prior to ATR-FTIR analysis. The presence of an adsorbed nanoparticle layer on the gel surface which persists after soaking enables to select the appropriate nanoparticles to use as adhesive. Conversely, the absence of nanoparticle at the gel surface implies weak adhesive properties of the tested nanoparticles.

Alternately, a second method rests on Scanning Electron Microscopy (SEM) in combination with Energy Dispersive X-ray (EDX). The sample preparation is identical to the one disclosed above for the ATR-FTIR method. EDX is an analytical technique used for the elemental analysis or chemical characterization of a sample. The first micrometers of the surface are probed.

Finally, with the method of thermal isotherms (Hourdet, D. and L. Petit (2010). Macromolecular Symposia. C. S. Patrickios. Weinheim, Wiley-V C H Verlag Gmbh. 291-292: 144-158.) the skilled professional can also determine comparatively the nanoparticles best suited for providing adhesion to a gel surface or can carry out thermal isotherms adjusting pH to optimize adsorption mechanism, for example for anionic polyacrylic acid (PAA) macromolecules on the silica surface (Wisniewska, M. (2010) Journal of Thermal Analysis and Calorimetry, 101(2), 753-760. doi:10.1007/s10973-010-0888-4).

The method consists in preparing different series of mixtures of nanoparticle and polymer chains (of the same chemical nature as the gel) by introducing increasing amounts of polymer into the nanoparticle suspension of fixed concentration. The samples are then stored for several days to provide sufficient time to the polymer chains to be adsorbed onto the nanoparticles surface and to equilibrate. The nanoparticles are then settled down by centrifugation and the supernatant is recovered for titration. The total concentration of free polymer chains in the supernatant ($Cp$) is determined by titration using GPC (Gel Permeation Chromatography) or using a total organic carbon (TOC) analyser or other methods of titrations. The amount of adsorbed polymer on nanoparticle surfaces ($\Gamma$ in mg/m$^2$) can be estimated and is usually plotted against the equilibrium polymer concentration.

Advantageously, the nanoparticles are used as an aqueous suspension (or dispersion) of nanoparticles.

According to another embodiment, the compositions of nanoparticles are under powder form.

Aqueous suspensions of nanoparticles are commercially available. One can mention the aqueous suspensions of colloidal silica Ludox® from Grace Davison.

They can be prepared for any of the above-mentioned material by using methods known to the skilled professional Stöber et al. method (Controlled growth of monodisperse silica spheres in the micron size range, Journal of colloid and interface science (1968)).

Advantageously, the aqueous suspension of nanoparticles which can be used according to the invention does not contain any other gluing agent. It means that the aqueous suspension of nanoparticles does not contain a compound known as a gluing agent in a concentration that would allow it to play the function of gluing agent. Among known gluing agents, one can mention:

- Synthetic adhesives: monomers, synthetic polymers (other than polymer nanoparticles), notably cyanoacrylates, urethanes, dendrimers;
- Natural adhesives: fibrin, collagen, gelatin, polysaccharides.

Preferably, the composition, preferably the aqueous suspension, of nanoparticles contains no more than 20%, or better, no more than 10% by weight of other gluing agent as compared to the weight of the dry matter of the composition, respectively aqueous suspension, preferably, less than 5% weight, even more preferably less than 2 % and better less than 1%, even better, less than 0,5%.

However, materials distinct from the nanoparticles can be present in the composition, preferably, the aqueous suspension, and notably mineral or organic ions can be present in the composition, preferably the suspension.

According to the invention, the nanoparticles have the function of gluing agent in the compositions wherein they are present. And in these compositions, the nanoparticles represent from 10 to 100% by weight of the weight of the dry matter of the aqueous suspension.

Preferably, the nanoparticles represent from 20 to 100% by weight of the weight of the dry matter of the composition, preferably the aqueous suspension, even more preferably, from 30 to 100%, and advantageously, from 40 to 100%, better from 50 to 100%, even better from 60 to 100%, preferably from 70 to 100%, even better from 80 to 100%, even more preferably from 90 to 100%. According to a favorite variant, the nanoparticles represent from 95 to 100% by weight of the weight of the dry matter of the composition, preferably the aqueous suspension, even better from 98 to 100%, and even more preferably from 99 to 100%.

Preferably, the aqueous suspension of nanoparticles consists essentially of nanoparticles suspended in water. It means that other components can be present in the suspension, but they do not modify the properties of the suspension in a noticeable manner. Especially, other components can be present in the suspension, but they do not significantly modify the adhesive properties of the suspension.

Alternately, the composition of nanoparticles consists essentially of nanoparticles in powder form. It means that other components can be present in the powder, but they do not modify the properties of the powder in a noticeable manner. Especially, other components can be present in the powder, but they do not significantly modify the adhesive properties of the composition.

Among components that can be present in the aqueous suspension of nanoparticles, one can mention: mineral or organic ions, small organic molecules, proteins, physiological fluids. Notably, such components can be anti infectives, anti bacterians, preservatives, antibiotics, PEG, polymers of varied nature...

Concentrations are adjusted to obtain suitable viscosities for application. Alternately, powders can also directly adsorb to the surface of a hydrogel.

In general suspensions of viscosity from about 10 Pa.s or less are used, preferably lower viscosities ($10^{-3}$ Pa.s). For non-spherical particles, like particles of CNT, or CNC type, the concentration is adjusted so that the viscosity remains fairly low.

The pH of the aqueous suspension of nanoparticles can be of any value from 1 to 14 and is adapted according to the application. pH can be adjusted to optimize adsorption, for example for anionic polyacrylic acid (PAA) macromolecules on the silica surface (Wisniewska, M. (2010), Journal of Thermal Analysis and Calorimetry, 101(2), 753-760. doi:10.1007/s10973-010-0888-4) but also to keep the stability of the nanoparticles composition. For polyelectrolyte or amphoteric gels, the pH of the nanoparticles composition is adjusted to obtain nanoparticles of charges opposed to gel's charges.

- The hydrogel:

Gels are solid, jelly-like materials based on a dilute cross-linked structure within a fluid. Gels do not flow in the steady-state. The major part of a gel is a liquid, yet they behave like solids due to their three-dimensional cross-linked network structure within the liquid. The crosslinked structure can be based on polymers of any nature or on surfactant molecules.

A hydrogel, also named a colloidal gel, is based on a network of chains, generally of the polymer type, in which water is the dispersion medium. Hydrogels can contain over 99.9% water and can be based on natural or synthetic polymers.

Hydrogels have varied properties, among which one can mention:

Hydrogels are rich in water, yet they can be manipulated. They are conformable. Hydrogels have the capacity to create or maintain a moist environment

Some hydrogels are sensitive to external stimuli, which are also known as 'Smart Gels' or 'Intelligent Gels'. These hydrogels have the ability to sense changes of pH, temperature, electrical current, or the concentration of a molecule in their environment, like a metabolite. In reaction to such a change they can contract or swell or release their load as result of such a change. Hydrogels that are responsive to specific molecules, such as glucose or antigens, can be used as biosensors.

Hydrogels can comprise molecules dispersed in the gel, grafted on the chains which constitute the gel or solubilized in the aqueous medium. Notably culture media can be included in a gel, which can be used as a carrier for cell culture. Active principles can be included in a hydrogel which can be used for the controlled release of said actives. For example cosmetic or therapeutic active principles can be included in a hydrogel.

Hydrogels find use in numerous applications including:

- Microfluidic, wherein they can be used as valves;
- Cell culture: cell culture plaques can include hydrogel-coated wells, microbiochips can include gel coated locations for cell culture;
- Gel permeation, either classical gel permeation or microfluidic separation;
- Actuation;
- Tissue engineering: hydrogels can be used as scaffolds containing or supporting cells for tissue repairing;
- Sustained-release actives delivery, notably as cosmetic or pharmaceutical patch or dressing;
- Medical applications: biosensors, medical electrodes, contact lenses, tissue encapsulation;
- Surgery: as suturing strip, or to avoid rupture of amniotic membrane after amniocentesis;
- Food: jelly, tofu, some industrial cheeses and dairy products, surimi, are examples of mainly protein-based gels of animal or vegetal origin,
- Agrochemistry: they can be used as granules for holding soil moisture in arid areas and/or delivering nutrients to the soil.

All kinds of hydrogels can be concerned by the invention. More specifically, the invention is concerned by gluing hydrogels of synthetic or natural origins.

Among synthetic hydrogels, one can mention:

A hydrogel of a polymer selected from: PDMA (poly(N,N-dimethyl acrylamide)., poly-NIPAM (poly(N-isopropylacrylamide)), a synthetic or extracted gel based on proteins, like gelatin, or on polysaccharides, like agarose, methylcellulose, hyaluronan, chitosan...

Such synthetic gels can comprise other molecules, like active principals (cosmetic, pharmaceutics), nutrients, proteins, cells, physiological fluids...

In addition, many human or animal tissues are based on gel or gel-like materials and will be considered as hydrogels in the context of the invention. Among natural hydrogels which are concerned by the invention, one can mention biological tissues, notably viscera, like liver, kidneys, lungs, intestines but also blood vessels, muscles, tendon, cornea...

Favorite nanoparticles for gluing human or animal tissues are selected from: silica nanoparticles, magnetic nanoparticles (like iron oxides for medical diagnosis)

The invention is more specifically directed to the gluing of non self adhesive hydrogels. Self adhesion can be simply tested by pressing two pieces of a gel between two fingers and observing if the gel pieces adhere or separate when one part is lifted.

- The other article:

The other article which is used in the invention can be of any material to which it is desired to make a hydrogel adhere. The choice of materials for the other article is a function of the polarizability and materials on which the nanoparticles can be adsorbed. The other article can be of a material selected from: a hydrogel, a glass, a polymer, a biological tissue, a metal.

When the other article is a hydrogel, it can be identical to or different from the first hydrogel. For example, thanks to the gluing method according to the invention, one can make an assembly comprising a superposition of hydrogels of identical or similar chemical nature, presenting a gradient of a given property (concentration, pH, ionic strength). Alternately, one can make an assembly of gels of different chemical natures.

Among polymers to which a hydrogel can be glued, one can mention polycarbonate, polystyrene, polymethylmethacrylate, polypropylene, etc.

A hydrogel can be made to adhere to a glass thanks to the method disclosed here. This type of assembly is of interest in laboratory equipment like: microfluidics, biochips, gel permeation equipment...Preferably, before gluing the hydrogel, the glass is submitted to a treatment favoring the formation of reactive functions at its surface like a plasma treatment

Before gluing, the surfaces can be treated specifically by anti-microbial compounds, cationic compounds, depending on the pH and pKa of the nanoparticles composition, so that nanoparticles can be adsorbed.

A hydrogel can be made to adhere to a biological tissue, like skin. For example, a patch containing a cosmetic or therapeutic active in a hydrogel can be adhered to the skin by spreading an aqueous suspension of nanoparticles to the surface of the gel before applying it to the skin. It can thus provide a continuous or delayed, or prolonged release of the active principle. Medical electrodes of hydrogel material can also be adhered to the skin by this method. A provisional or permanent prosthetic device including a hydrogel can be adhered to any tissue, like a blood vessel, and nanoparticles can create sufficient adhesion to permit suturing and/or biological tissue auto-repairing. Tissue repair hydrogels can be adhered to damaged tissue, like burnt skin.

And the same method of gluing can be used to make a hydrogel adhere to a mechanical support (polymer film, non-woven polymer network for example) when manufacturing an article like a patch or a dressing, an implant, a contact lens, a medical electrode.

An adhesion between two parts of an organ of a gel or gel-like nature can be built thanks to nanoparticles suspension, for repairing damaged organs after an accident or during a surgical intervention. The organ can then self-repair thanks to cell colonization. The nanoparticles suspension can also be used for provisionally maintaining together two parts of an organ for suturing. Currently, damaged liver is repaired by wrapping the organ in a textile until self-repair is achieved. However, such a method presents a risk of infection due to the presence of the textile. Using a suspension of nanoparticles, notably a suspension of hydrocolloïdal silica as surgical glue presents numerous advantages: it is safe, cheap, efficient, easy to use.

- The method for gluing a hydrogel to an article:

The method according to the invention is remarkably simple: A composition of nanoparticles of the desired composition is prepared or a commercial composition is used. This composition of nanoparticles is applied on at least one face of the hydrogel. The face of the hydrogel on which the nanoparticles have been applied is applied to the article.

According to a favorite variant, the composition is an aqueous suspension of nanoparticles.

According to another embodiment, the composition is a powder composition.

For both synthetic and biological hydrogels, strong and rapid adhesion is achieved at room temperature by simply spreading a droplet of nanoparticle solution on the gel surface before bringing the two pieces into contact.

Alternately, the powder composition is spread at the surface by known means and the excess (non adsorbed) powder is removed.

Advantageously, the nanoparticles composition used in the method for gluing a hydrogel to another article presents the characteristics above disclosed.

The quantity of nanoparticles deposited at the surface of the hydrogel is advantageously from $0.1 mg/m^2$ to 10 $g/m^2$. Depending on the size of the nanoparticles, the coverage of the surface is to be adjusted. These values can be from 1 $mg/m^2$, preferably for small particles, and up to 0.2 $g/m^2$, preferably for large particles.

For large particles (typically of the order of 300 nm) the coverage is large, of the order of $4 g/m^2$. For particles of smaller size (diameter of about 2 nm) rates coverage is preferably of the order of $10 mg/m^2$.

Preferably, it is believed that optimum adhesion is obtained for a dense monolayer on the nanoparticles surface. The density of coverage can be evaluated on the assembly by ATR-FTIR or by SEM.

As illustrated in the experimental part, the inventors achieved strongly bonding together pieces of hydrogels, which have either the same or different chemical nature or rigidity.

Surprisingly, the step of contacting the hydrogel (already treated with the suspension of nanoparticles) with the other article, can be achieved under water. A good adhesion of the two elements is obtained according to this variant. For gels that are not at swelling equilibrium when the assembly is immersed in water, both the gels and bond layer swell.

To glue fully swollen gels, according to a favorite variant, the surface layer is just slightly dried before applying the nanoparticles composition. Alternately, fully swollen gel can be glued thanks to an appropriate choice of nanoparticles (size and affinity).

When the article or the hydrogel is selected from biological tissues *in vivo,* the method is a surgical method or a therapeutic method. It can be a method for repairing a damaged organ, or for adhering an implant, a prosthesis, a patch or a dressing to a biological tissue.

As disclosed in the experimental part, to illustrate the promise of the method for biological tissues, pieces of calf liver were glued to obtain, in a dozen of seconds, manipulable assemblies.

According to one embodiment, none of the article and the hydrogel is selected from biological tissues *in vivo.*

According to this embodiment the article or the hydrogel can independently be selected from or include *ex vivo* or *in vitro* isolated or cultured biological tissues. The article or the hydrogel can independently also be of a synthetic material or a natural material of non human, and non animal origin.

- Assembly of a hydrogel and an article :

The invention is also directed to an assembly of at least one hydrogel and at least one other article, wherein the interface between the at least one hydrogel and the at least one other article is a layer of nanoparticles. In the assembly according to the invention, in case the at least one hydrogel or the at least one other article is a biological tissue, it is an isolated tissue or a cultured tissue.

In the assembly, the concentration of nanoparticles at the interface is preferably from 0,1 mg/m$^2$ to 10 g/m$^2$, advantageously from 1 mg/m$^2$, preferably for small particles, and up to 0.2 g/m$^2$, preferably for large particles

Such an assembly can in a non limitative manner be selected from: a microfluidic equipment, a biochip, a gel permeation equipment, an actuation gel assembly, a food gel assembly, a cosmetic or pharmaceutical patch or dressing, a biosensor, a medical electrode, a contact lens, an implant, a prosthesis.

The invention is remarkable in that the adhesion between the hydrogel and the article is resistant to humidity. Glued assemblies can resist immersion and swelling in water.

The preparation of an assembly according to the invention is illustrated in figure 1. Figure 1a: two hydrogels (1) and (2) are placed opposite with a layer of nanoparticles (3) in between. The two hydrogels are pressed and both are in contact with the nanoparticles (3). In the adhesive layer, nanoparticles (3) act as connectors between gel pieces (1) and (2) and gel-chains (1.1) (2.1) bridge different particles (Fig. 1b).

- Kits:

The invention is also directed to a kit for gluing a hydrogel to an article, wherein said kit comprises at least a hydrogel and at least a composition, preferably an aqueous suspension, of nanoparticles.

Advantageously, the nanoparticles suspension used in the method for gluing a hydrogel to another article presents the characteristics above disclosed.

Alternately, the nanoparticles composition is a nanoparticles powder composition.

Said kit can comprise two or more compartments for separately conditioning the hydrogel and the composition of nanoparticles and for permitting an optimized use thereof. For example the kit can comprise a collection of hydrogels packaged in independent compartments and a flask comprising the suspension of nanoparticles with an appropriate distribution means (dripper, spray or brush for example). Alternately, it can comprise one hydrogel and the appropriate quantity of nanoparticles suspension for gluing said gel. In said kit, the hydrogel can be part of a more complex article. One example is a kit wherein the hydrogel is part of a cosmetic or dermatological patch, or a dressing.

According to a variant, the invention is also directed to a surgical kit, wherein it comprises at least one composition of nanoparticles, preferably consisting essentially of a suspension of nanoparticles, and at least one article chosen from: a suturing needle, a suturing strip, suturing staples, a prosthesis, an implant. The two parts are conditioned in sterile containers. They are complementary, since the suspension of nanoparticles can be used to glue in a provisional manner two parts of an organ, or an organ and a medical device (a prosthesis for example). Said gluing makes suturing by a known method easier. Said surgical kit can comprise any other article of use, like a hydrogel for example.

The invention has been described with reference to preferred embodiments. However, many variations are possible within the scope of the invention.

- Experimental part:

I- Methods summary

- Nanoparticles suspension:

Silica Ludox® TM-50, HS-40, and SM-30 water solutions with, respectively, concentration of 52, 40, and 30 wt% at pH 9, 9.5 and 10, Si02/Na20 ratio of 200-250, 89-101, and 45-56, radii of about 15, 9, and 5 nm, were purchased from Aldrich and used as received. Silica particles AL-30 with radius of about 50 nm were synthesized via Stöber et al. method (Controlled growth of monodisperse silica spheres in the micron size range. Journal of colloid and interface science (1968)) and dissolved in water at 30 wt% (pH=8.5). Multi-wall carbon nanotubes (CNT) were supplied by Arkema (Graphistrength® C100) and purified with sulphuric acid. Thymine-grafted CNT particles (CNT-Thy) were synthesized via method of Prevoteau, A., Soulié-Ziakovic, C. & Leibler, L. Universally Dispersible Carbon Nanotubes. J. Am. Chem. Soc. 134, 19961-19964 (2012). Cellulose nanocrystals (CNC1) bearing sulfate and hydroxyl groups have been prepared via method of Bondeson, D., Mathew, A. & Oksman, K. Optimization of the isolation of nanocrystals from microcrystalline cellulose by acid hydrolysis. *Cellulose* **13**, 171-180 (2006). Cellulose nanocrystals CNC2 were prepared using the same method, but replacing sulfuric by chlorhydric acid. The suspensions diluted to desired concentration (0.5 and 3% wt% for CNTs and CNCs, respectively) were sonicated for 30min just before use.

- Hydrogel:

PDMA and PDMA nanocomposite gels were prepared via method of Carlsson, L., Rose, S., Hourdet, D. & Marcellan, A. Nano-hybrid self-crosslinked PDMA/silica hydrogels. Soft Matter 6, 3619-3631 (2010). Polyacrylamide hydrogels were prepared by *in -situ* free radical polymerization of acrylamide using thermal dissociation of potassium persulfate (KPS) as initiator, at 80°C. *N,N'*-methylenebisacrylamide (MBA) was used as cross-linker and MBA/DMA ratio was 0.1, 0.5, 1 and 1.5 mol.%, for samples 50.1, 50.5, S1.0 and S1.5, respectively and MBA/AAm was 0.1 mol.% for A0.1 gel. At the preparation state, gels matrix hydration was fixed at 87.7 wt%. To avoid network defects that lead to a very weak self-adhesion of gels it is important to conduct synthesis under nitrogen conditions. Gelatine (Technical 1, VVR) gels were prepared by cooling, from 50°C to RT, 23 wt% aqueous solutions. We synthesized elastic PDMA gels with large range of cross-link densities and elastic moduli (Table 1).

Table 1A: Properties of PDMA and gelatin gels

| Sample | Preparation state | | | | | | | |
|---|---|---|---|---|---|---|---|---|
| | water (g) | MA (g) | AAm (g) | MBA (mg) | Silica vol. fraction | Gelatine (g) | Q | E (kPa) |
| S0.1 | 10.62 | 1.485 | | 2.3 | | | 8.5 | 10 ± 2 |
| NC | 10.62 | 1.485 | | 2.3 | 0.21 | | 8.5 | 93 ± 8 |
| S0.5 | 10.69 | 1.485 | | 11.6 | | | 8.5 | 26 ± 1 |
| S1.0 | 10.77 | 1.485 | | 23.0 | | | 8.5 | 45 ± 2 |
| S1.5 | 10.94 | 1.485 | | 46.2 | | | 8.5 | 60 ± 4 |
| A0.1 | 7.62 | | 1.065 | 2.3 | | | 9 | 11 ± 1 |
| Gelatine | 11.1 | | | | | 3.31 | 5.3 | |

Table 1B: Properties of PDMA and gelatin gels

| Sample | Swelling equilibrium conditions | | |
|---|---|---|---|
| | Q | E (kPa) | Swelling ratio $Q_e/Q_0$ |
| S0.1 | 41 ± 1 | 4 ± 1 | 5.1 |
| N | 29 ± 4 | 7 ± 1 | 3.4 |
| S0.5 | 23 ± 3 | -- | 2.7 |
| S1.0 | 17 ± 2 | -- | 2.0 |
| S1.5 | 15 ± 2 | -- | 1.7 |
| A0.1 | | | |
| Gelatine | 29±2 | | 5.5 |

Swelling degree Q is defined as the ratio of hydrated gel volume to the dry polymer volume. *E* denotes the tensile modulus at the preparation state, $Q_0$ or at the swelling equilibrium conditions, $Q_e$, respectively. For the synthesis of PDMA gels, 0.041g of potassium persulfate (KPS) and 22.5 µL of *N,N,N',N'*-tetramethylethylenediamine TEMED were introduced as redox initiators. For the preparation of polyacrylamide gels: two aqueous solutions were prepared: KPS at 4.5wt% and MBA at 1.2 wt%. First, MBA solution and AAm were dissolved at 25°C in water. The KPS solution was added and the homogeneous solution was purged with nitrogen during 15 minutes under magnetic stirring. The mixture was finally transferred, under nitrogen atmosphere, into laboratory-made moulds previously sealed and put under nitrogen atmosphere. The sealed moulds were placed in an oven at 80°C for 24 hours. Gelatine gels were prepared by dissolving the gelatine powder in deionized water under stirring at 50°C during two hours. The gelatine concentration was 23 wt.%. The mixture was then introduced in moulds and left at room temperature during 30 minutes. Moulds were stored at 6°C during two days prior testing and left at room temperature for 1 h before testing.

- Testing methods:

Lap joint geometry: Displacement was measured by a video extensometer that followed two markers (white dots), which were placed at 5 mm distance from the edge of the lap joint. The total length of assembled ribbons was 40 mm. *w* denotes the width and *h* the thickness of gel ribbons. *l* is the overlap length.

Scanning electron micrographs were obtained using a Field Emission SEM (Hitachi SU-70).

Lap-shear and mechanical tests were performed on an Instron 5565 tensile testing machine equipped with a 10N load cell and an optical extensometer, at a speed of 150 mm/min. For mechanical tests PDMA gel samples were cut into rectangular ribbon shape of 50 mm x 5 mm x 2 mm. Single lap-shear geometry was used with a joint of 10 mm length by 5 mm width. Extensometer markers were placed 5 mm from the edge of the lap joint. The initial distance between markers was about 20 mm for gels at preparation conditions $Q_0$ and correspondingly larger for gels at $Q_e$. The total length of the assembled ribbons was 40 mm. In order to avoid systematic failure in the vicinity of the clamps, gelatine samples had a dog-bone shape following the ISO4661-1 standard with the reduced section of samples of 25 mm x 4 mm x 2 mm. Lap joint dimensions were 10mm x 4mm.

When interfacial failure occurs by peeling, from the measured adhesive failure force *F* the adhesion energy can be calculated (Kendall, K. Molecular Adhesion and Its Applications. (Plenum Publishing Corporation, 2001; Kendall, K. Cracking of Short Lap Joints. The Journal of Adhesion 7, 137-140 (1975)), $G_{adh}=3(F/w)^2/(2Eh)$, where *w* and *h* denote, respectively, the width and thickness of the ribbon, and *E* is the tensile modulus.

Fracture tests were performed using the classical single edge notch geometry using the Instron machine. For that purpose, a cut was made in the centre of the rectangular samples using a blade. Each notch was measured by optical microscopy in order to determine its exact length (*c*), approximately 1 mm. The same procedure as the one used for tensile tests was performed: the strain rate was fixed to be 0.06 s-1, the force and the displacement data were recorded. In the case of a single edge notched specimen, Rivlin, Thomas and Greensmith determined the fracture energy as:

$$G_{IC} = 2KW(\lambda_c)c$$

with *W* the strain energy density, $\lambda_c$ the extension ratio at break and *c* the initial length of the crack. (Rivlin, R.S., Large Elastic Deformations of Isotropic Materials .4. Further Developments of the General Theory. Philosophical Transactions of the Royal Society of London Series a-Mathematical and Physical Sciences, 1948. 241(835): p. 379-397 ; Greensmith, H.W., Rupture of rubber. X The change in stored energy on making a small cut in a test piece held in simple extension. Journal of Applied Polymer Science, 1963. 7(3): p. 993-1002.)

Thermal isotherms are determined by the method of Hourdet, D. and L. Petit (2010). Macromolecular Symposia. C. S. Patrickios. Weinheim, Wiley-V C H Verlag Gmbh. 291-292: 144-158. Different series of mixtures of nanoparticle and polymer chains (of the same chemical nature as the gel) were prepared by introducing increasing amounts of polymer (N-acrylamide, PNIPA, PEO) into the nanoparticle suspension Ludox TM-50. The samples are then stored for several days to provide sufficient time to the polymer chains to be adsorbed onto the nanoparticle surface and to equilibrate. The nanoparticles are then settled down by centrifugation and the supernatant is recovered for titration. The total concentration of free polymer chains in the supernatant (Cp) was determined by titration using GPC (Gel Permeation Chromatography) or using a total organic carbon (TOC) analyser or other methods of titrations. The amount of adsorbed polymer on nanoparticle surfaces ($\Gamma$ in mg/m$^2$) is estimated and is plotted against the equilibrium polymer concentration.

The isotherms illustrated in Hourdet et al.immediately give evidence that both PDMA and PNIPA interact more energetically than PEO with silica surfaces. In the case of PDMA and PNIPA, a strong adsorption regime is observed at low coverage of the particles, with a sharp increase of the adsorbed amount of polymer for $\Gamma/\Gamma_{max}<0.5$ ($\Gamma<0.5$-0.7 mg/m$^2$). This regime is followed by a weaker interaction domain ($\Gamma/\Gamma_{max}>0.5$) and ends up at the plateau value, which is approximately the same for the two polymers: $\Gamma_{max} \cong 1$ mg/m$^2$. The same holds for PEO chains but with a smaller extent as the plateau value is reached at $\Gamma_{max} \cong 0.6$ mg/m$^2$. Applying the Langmuir isotherm model in the high coverage

regime ($\Gamma/\Gamma_{max}>0.5$, Figure 1), the adsorption equilibrium constant (K) can be estimated and is seen to increase from PEO to PNIPA and then to PDMA: K=3, 6 and 30 L.g$^{-1}$, respectively.

From these results, we can anticipate that silica nanoparticle solutions enable the gluing of PDMA and PNIPAM gels. But silica NP can also be modified to adsorb onto poly(acrylamide) gels and to provide adhesive properties to the poly(acrylamide) gel (Pefferkorn, E., A. Carroy, et al. (1985). Macromolecules 18(11): 2252-2258.) or pH can be adjusted to optimize adsorption, for example for anionic polyacrylic acid (PAA) macromolecules on the silica surface (Wisniewska, M. (2010). Temperature effect on adsorption properties of silica-polyacrylic acid interface. Journal of Thermal Analysis and Calorimetry, 101(2), 753-760. doi:10.1007/s10973-010-0888-4)

Fourier transform infrared spectroscopy (FTIR) coupled with Attenuated total reflectance (ATR)

Ludox TM-50 shows good adhesive properties with 50.1 gels at preparation state, $Q_0$. On the other hand, very weak bonding properties are obtained when the substrate is at its maximum swelling equilibrium, $Q_e$.

ATR-FTIR was used in order to compare the adsorption properties of Ludox TM-50 on 50.1 at $Q_0$ and on 50.1 at $Q_e$. For that purpose, two samples of 50.1 gels containing the same polymer weight were used, for one at $Q_0$ and for the other one at $Q_e$. Both samples were immersed in Ludox TM-50 solution for 30 seconds then soaked in a large volume of deionized water during 3 days, exchanging the solvent every 12 hours. Prior to ATR-FTIR analysis the swollen 50.1 samples were dried (at 80°C for 2 days).

Fourier transform infrared spectroscopy was carried out using a Bruker TENSOR TM27 spectrometer fitted with a diamond ATR accessory. Figure 13 shows the ATR-FTIR spectra of the surfaces of S0.1 gel submitted to this preparation process. Presence of silica nanoparticle is tracked by the 1100 cm$^{-1}$ band and reveals that adsorption is weaker when the 50.1 gel is at its maximum swelling equilibrium. Adhesive properties confirm this observation. Silica band assignment is given from Parida et al. (Parida SK, Dash S, Patel S, and Mishra BK. Advances in Colloid and Interface Science 2006;121(1-3):77-110.) and references wherein: 800 cm$^{-1}$ $\delta$(OH) silanol ; 975 cm$^{-1}$ $\nu$(Si-OH) ; 1100 cm$^{-1}$ $\nu_{\alpha s}$(Si-O-Si) 1630 cm$^{-1}$ $\delta$(O-H) molecular water. Gel spectra were normalized using the 1400 cm$^{-1}$ band assigned to $\delta_s$(CH$_3$) of poly(dimethylacrylamide) hydrogel (Sekine Y and Ikeda-Fukazawa T. Journal of Chemical Physics 2009;130(3).) and references wherein $\nu$ is the stretching and $\delta$ is the bending mode.

Scanning Electron Microscopy (SEM) in combination with Energy Dispersive X-ray (EDX) spectroscopy.

EDX is an analytical technique used for the elemental analysis or chemical characterization of a sample. The first micrometers of the surface are probed. The sample preparation procedure is the same as for the ATR-FTIR test. Figure 14a and 14b compare the morphology and element analysis of the surfaces of S0.1 gel: 50.1 at Q0 (a) and 50.1 at Qe (b) prepared according to the above disclosed protocol. SEM micrographs and EDX results confirms that silica adsorption is weaker for 50.1 at Qe.

II- Experiments

Water soluble polymers from substituted acrylamides such as poly(dimethylacrylamide) (PDMA) or poly(n-iso propyl acrylamide), readily adsorb to silica nanoparticles (Hourdet, D. & Petit, L., Macromol. Symp. 291-292, 144-158 (2010)).

- Example 1:

To demonstrate the concept and test the importance of gel chain adsorption onto particles, we synthesised hydrogels, S0.1, made of poly(dimethylacrylamide) (PDMA) and A0.1, made of poly(acrylamide) (PAAm). Both gels had the same cross-linking density, 0.1 mole% and contained 88 wt% of water (Table 1). Poly(acrylamide) chains do not adsorb onto silica (Griot, O. & Kitchener, J. A. Role of surface silanol groups in the flocculation of silica suspensions by polyacrylamide. Part 1. Chemistry of the adsorption process. Trans. Faraday Soc. 61, 1026 (1965)), whereas poly(dimethylacrylamide) adsorbs readily (Hourdet, D. & Petit, L. Hybrid Hydrogels: Macromolecular Assemblies through Inorganic Cross-Linkers. Macromol. Symp. 291-292, 144-158 (2010)). Both PDMA and PAAm gels did not adhere to themselves. When a 15 $\mu$L drop of TM-50 silica suspension was spread on PDMA gel surface and another PDMA piece was pressed to form a lap-junction, a strong adhesion was observed after few seconds of contact. In contrast, for poly(acrylamide) gels, even when we pressed hardly and for a very long time, we could not make lap junctions that held under their own weigh and thus confirmed that the lack of gel chains adsorption onto nanoparticles prevents gluing.

But silica NP can be modified to adsorb onto poly(acrylamide) gels and provide adhesive properties to the poly(acrylamide) gel (Pefferkorn, E., A. Carroy, et al. (1985). Macromolecules 18(11): 2252-2258.). For example, the silica surface is firstly made hydrophilic by soaking for 24 h in hot hydrochloric acid medium and then treated with aluminum chloride in chloroform in order to replace some silanols (SiOH) by aluminum chloride groups, which by hydrolysis of the latter are converted to aluminol (AlOH).

We found that for all lap joints with large overlap length *l* (between 4 and 20 mm) the failure systematically occurred outside the bonding junction, showing that the joint was stronger than the gel itself (Fig. 9a, 2a, 4). Lap joints with small overlap length or which are made of narrow and thick gel ribbons become distorted under tensile load and interfacial failure by peeling can occur. From measured adhesive failure force $F$ (Fig. 9a) we can evaluate the adhesion energy (Kendall, K. Molecular Adhesion and Its Applications. (Plenum Publishing Corporation, 2001; Kendall, K. Cracking of Short Lap Joints. The Journal of Adhesion 7, 137-140 (1975)), $G_{adh}=3(F/w)^2/(2Eh)$, where $w$ and $h$ denote, respectively, the width and thickness of the ribbon, and $E$ is the tensile modulus. We found $G_{adh}$ to be $6.6\pm1.6$ J/m$^2$ and $6.2\pm1.4$ J/m$^2$, respectively, for short ($l$=2mm, $w$=5mm, $h$=2mm) and narrow and thick ($l$=5mm, $w$=2mm, $h$=5mm) joints (Fig. 2b).

- Example 2:

To probe how the size of silica particles affects the adhesion, failure force $F$ was measured in a lap shear tensile test with geometry (w=5mm, h=2mm, 1=5mm) that offers a good compromise between adhesive joint weakness and measurement precision (Fig. 9b, Fig. 6). In this geometry adhesive failure by peeling was observed for junctions glued with smaller particles (SM-30 and HS-40 with radii 5 and 9 nm, respectively) and bulk fracture outside the junction for bigger particles (TM-50 and AL-30 with radii 15 and 50 nm, respectively). Using AL-30 particles led to bulk failure even when the joints were very short, narrow and thick. To induce peeling making cuts at interface was necessary. Thus, it seems that adhesion, strong in all cases, increased when particle size was increased, although it should be noted that changing the size of particles implies some variations of the surface chemistry as well.

- Example 3 :

Particle surface chemistry can be harnessed to bring adhesion by improving suspension stability or by promoting specific interactions such as hydrogen bonding that strengthen the particle adsorption to gel surface. Thus grafting thymine to carbon nanotubes brought adhesion. Similarly, cellulose nanocrystals CNC1 bearing sulfate groups yield adhesion strength comparable with that obtained with nanosilica whereas CNC2 particles with hydroxyl groups only were useless as a glue for 50.1 gels (Fig. 9b).

- Example 4:

Gluing strength also depends on gel properties. In tightly cross-linked gels, strands are more constrained and there is a higher entropy penalty for adsorption (Johner, A. & Joanny, J.-F. Adsorption of polymeric brushes: Bridging. J. Chem.Phys. 96, 6257 (1992)). In addition, energy dissipation mechanisms discussed above are less efficient for short strands $N$. Adhesion strength is therefore expected to be weaker for more rigid gels. Figure 9b shows that indeed adhesion energy $Gadh$ decreased by a factor of 3 when the crosslinking degree of bonded gels was increased by a factor of 10 corresponding to increase of tensile modulus by a factor of 4.5. At the same time, more rigid gels dissipate less energy during fracture and are more brittle as evidenced by single edge notched tensile test results (Fig. 9b). Hence, in practice, even for rigid gels, gluing by nanoparticles is sufficiently strong to allow designing of joints that withstand tensile stresses without adhesive failure (Fig. 3a, 3b, 3c). It is also possible to glue strongly gels with very different rigidities (Fig. 7a).

- Example 5:

Gluing by nanoparticle solutions is not limited to hydrogels that are in the assynthesized state. Dehydration of gels before gluing is expected to increase adhesive strength, whereas gel swelling should have the opposite effect. Indeed, in a more swollen state strands are more stretched and adsorb less readily. Choosing particles with right size and affinity to gels becomes important. We used AL-30 silica solution to glue swelled S01 gel ribbons and measured $Gadh$ as a function of swelling degree $Q$ (Fig. 4e). Although lower by a factor of 2.4 when compared with gels at $Q$=16, the adhesion energy at the maximum (equilibrium) swelling $Qe$=41 (97,6 v/v% of water) was as high as $1.6\pm0.6$ J/m$^2$.

- Example 6:

The design principle assures that adhesion remains when the joint is immersed in excess solvent and swells since particles once adsorbed stay strongly anchored to the gel surfaces. Indeed, the probability of total desorption of a gel strand is exponentially small $\exp(-n\varepsilon/kT)$ (Sprakel, J., van der Gucht, J., Cohen Stuart, M. A. & Besseling, N. A. M. Rouse, dynamics of colloids bound to polymer networks. Phys. Rev. Lett. 99, 208301 (2007) ; Montarnal, D., Capelot, M., Tournilhac, F. & Leibler, L. Silica-Like Malleable Materials from Permanent Organic Networks. Science 334, 965-968 (2011) ; Gent, A. N., Hamed, G. R. & Hung, W. J. Adhesion of elastomer layers to an interposed layer of filler particles. The Journal of Adhesion 79, 905-913 (2003). To confirm strong anchoring, we spread a droplet of TM-50 silica solution on the gel surface and washed the surface in pure water several times. Scanning electron microscopy showed that nanoparticles densely cover the surface, even after washing (Fig. 5b). We then glued S01 hydrogels having swelling degree of $Q0$=8.5 with TM-50 nanoparticles and immersed lap joint in water to reach the maximum, equilibrium swelling of $Qe$=41. The junction withstood five-fold volume increase and adhesion energy measured in lap-shear test was $1.8\pm0.5$ J/m2, i.e. 3.5 times lower than in preparation state (Fig. 5d, 4a). In the fully swollen state adhesion strength is weaker because detaching adsorbed chains is easier as adsorbed chains are already under higher swelling tension. Moreover, once detached the swollen strand is less prone to adsorb and strand-strand exchange dissipation processes are hindered.

Example 7:

Strong irreversible anchoring of once adsorbed particles brings an attractive possibility of self-repairing and/or re-positioning adhesive joints. Figure 5d shows that a joint, which was peeled, can recover its initial strength when ribbons are brought into contact and pressed with fingers for few seconds without any need to re-apply the glue (Fig. 8, 5d).

- Example 8:

Particle solutions offer simple method of gluing gels of different chemical nature provided particle surface chemistry is properly adjusted to allow adsorption on both gels. For example, using TM-50 silica solution a robust assembly of S01 and gelatin was achieved (Fig. 7b). Gelatine/PDMA 50.1 junction glued with TM-50 silica was immersed in water for one week to reach maximum equilibrium swelling did not break. Swelling ratios of gelatin and 50.1 gels are comparable (Qe/Q0=5) although their moduli are very different. Because of the stiffness mismatch the joined gels deform and curve under swelling, but the interface holds.

In many applications such as actuation, gluing gels of different rather than identical chemical nature presents advantages, including the possibility of assembling gels with different rigidity, but similar equilibrium swelling. Such assemblies can withstand equilibrium (maximum) swelling in excess water (Fig 12). In contrast, swelling of a glued assembly of chemically identical gels with mismatched swelling capacities can lead to high, heterogeneous osmotic stresses near the interface and produce slow interfacial failure. Glued at their preparation state by TM-50 solution, both PDMA 50.1 and PDMA S1.5 gels had initially the same size (diameter of about 10 mm). After 5 hours of swelling in deionised water, highly cross-linked PDMA S1.5 gel is less swollen than PDMA 50.1 gel Gel S01 shows a fivefold over-swelling when immersed in water, whereas a more tightly cross-linked gel S1.5 over-swells by a factor of 1.7 only. As a result, interfacial stresses induced by heterogeneous over-swelling exceed considerably shear stresses applied in mechanical lap-shear tests. Hence, for S0.1/S1.5 assemblies, interfacial failure was observed during immersion and over-swelling in water. Still adhesion joint held for quite a long time and the de-bonding was slow.

- Example 9A:

Soft biological tissues although incomparably more complex, mechanically and osmotically, resemble gels in many respects. To test gluing potentialities of nanoparticle solutions we cut from calf liver two ribbons $45\times18\times3$ mm$^3$. Cut pieces do not adhere to each other and cannot be glued by water at pH 9. We spread $60\mu$L of silica TM-50 solution on cut surface (without any pre-treatment or special drying) to make a lap joint with overlap length $l$=20 mm. After being pressed for 30 seconds with a finger (contact pressure of about $10^5$ Pa), the lap joint held strongly and could be manipulated with ease.

Normalized force-displacement curves are illustrated in figure 10. Lap shear adhesion tests for two different calf livers yield adhesion energy $Gadh\approx25\pm5$ J/m$^2$ and $Gadh\approx6\pm1.6$ J/m$^2$. The moduli of two livers are respectively $15.0\pm1.7$ kPa and $12\pm1.5$ kPa.

Example 9B:

Nanoparticles suspensions provide a simple method for gluing biological tissues with gels. For example, Ludox TM-50 was used to glue calf liver to gelatin gel. We cut from calf liver one ribbon of $45\times25\times3$ mm$^3$ and a gelatin sample was cut into rectangular shape of $15\times20\times3$ mm$^3$. The gelatin sample was soaked in TM-50 silica solution for 30 seconds then brought into contact and pressed to the calf liver surface for 30 seconds. The obtained gelatin/liver assembly can sustain handling and immersion in deionized water for several hours.

Example 10: Study of PDMA 50.1 gels gluing and influence of the volume of nanoparticles suspensions deposited

Silica solution Ludox TM-50 is used. Different volumes of silica are deposited on a junction of length = 15 mm, w = 5 mm in width and h = 2 mm thickness. A pressure of 10 kPa is exerted for 30 seconds. The joint is tested in the geometry of overlay covering.

We obtain the results illustrated in Figure 15, which shows that, whatever the volume of Ludox TM-50 deposited, cohesive failure is observed: there is a break out of the assembly, close to the junction.

Example 11: Use of nanoparticles suspensions for gluing gels to rigid surfaces - Solid substrates: Commercially available Polystyrene (PS), Polycarbonate (PC), Polymethylmethacrylate (PMMA), Polypropylene (PP) and glass solid surfaces were used. Samples were cleaned prior gluing with ethanol and rinsed with deionized water. Prior gluing, the samples were sonicated in deionized water for 3 minutes.

Example 11 A:

Nanoparticle suspensions can readily achieve underwater gluing onto rigid substrates. For example, using Ludox TM-50 silica suspensions a robust anchorage of gelatin gel onto a rigid substrate was obtained even underwater conditions. Rigid surfaces of Polystyrene (PS), Polycarbonate (PC), Polymethylmethacrylate (PMMA), Polypropylene (PP) and glass were individually placed in beakers filled with deionized water. Gelatin gels were cut into rectangular shape of 15 mm x 20 mm x 3 mm. Gelatin samples were brought into contact and pressed to the immersed solid surfaces. Absolutely no adhesion to any of these surfaces was observed. Gelatin gels immediately slipped from the solid surface. When the gelatin sample was soaked in TM-50 silica suspension for 30 seconds prior ensuring the contact between the gel and solid surface, efficient adhesion was observed only after few seconds of contact. The assemblies can be easily handled and the bond held well. The assembly can resist also immersion in deionized water. For example, after being immersed in deionized water for 24h, the gelatin/glass assembly showed strong adhesion.

Example 11B:

Nanoparticle suspensions enable gluing of biological tissue to solid surfaces. For example, Ludox TM-50 was used to glue calf liver to glass. We cut from calf liver one ribbon of 45x25x3 mm$^3$. Calf liver readily slipped from the glass surface. When the calf liver sample was soaked in TM-50 silica suspensions for 30 seconds prior ensuring the contact between the calf liver and the glass surface, efficient adhesion was observed only after few seconds of contact. Assembly can sustain handling and immersion in deionized water for several hours.

Conclusion:

The results suggest that nanoparticles solutions bring a way to simply assemble synthetic and biological hydrogels as well as biological tissues without affecting substantially the rigidity or permeability of the assembly. Powerful methods exist to tune and control surface chemistry of inorganic particles and latexes to achieve optimal particle adsorption and bonding. The possibility to self-repair and re-position peeled adhesive joints is an additional boon. Given the importance of wet adhesion in biomedicine and biotechnology as well as in more traditional coating and material technologies, our results open interesting avenues to develop new applications by simply assembling all kinds of chemically and mechanically mismatched tissues and/or gels.

**Claims**

1. A composition of nanoparticles for its use for gluing at least one hydrogel to at least one other article, wherein the nanoparticles represent from 10 to 100% by weight of the dry matter of the composition.

2. A composition of nanoparticles for its use according to claim 1, wherein it is an aqueous suspension of nanoparticles

3. A composition of nanoparticles for its use according to anyone of the preceding claims, wherein the nanoparticles are selected from: clays, silicates, alumina, silica, kaolin, grafted carbon nanotubes, grafted cellulose nanocrystals, hydroxyapatite, magnetic nanoparticles.

4. A composition of nanoparticles for its use according to anyone of the preceding claims, wherein the nanoparticles have an average particle size from 1 nm to 1000 nm, preferably from 2 nm to 500 nm even more preferably from 5 nm to 300 nm.

5. A composition of nanoparticles for its use according to anyone of the preceding claims, wherein the hydrogel is of a material selected from: PDMA, poly-NIPAM, a synthetic or extracted gel based on proteins, a synthetic or extracted gel based on polysaccharides.

6. A composition of nanoparticles for its use according to anyone of claims 1 to 4, wherein the hydrogel is a biological tissue.

**7.** A composition of nanoparticles for its use according to anyone of the preceding claims, wherein the other article is of a material selected from: a hydrogel, a glass, a polymer, a biological tissue.

**8.** An assembly of at least one hydrogel and at least one other article, wherein the interface between the at least one hydrogel and the at least one other article is a layer of nanoparticles, and wherein in case the at least one hydrogel or the at least one other article is a biological tissue, it is an isolated tissue or a cultured tissue.

**9.** An assembly according to claim 8, wherein the concentration of nanoparticles at the interface is from 0.1 mg/m$^2$ to 10 g/m$^2$.

**10.** An assembly according to claim 8 or claim 9, wherein the adhesion of the hydrogel to the other article is superior to the hydrogel's resistance and is superior to the other article's resistance.

**11.** An assembly according to any one of claims 8 to claim 10, wherein it is selected from: a microfluidic equipment, a biochip, a gel permeation equipment, an actuation gel assembly, an edible gel assembly, a cosmetic or pharmaceutical patch or dressing, a biosensor, a medical electrode, a contact lens, an implant, a prosthesis, a surgical strip.

**12.** A kit for gluing a hydrogel to an article, wherein said kit comprises at least a hydrogel and at least a composition of nanoparticles.

**13.** A surgical kit, wherein it comprises at least one composition of nanoparticles and at least one article chosen from: a suturing needle, a suturing strip, suturing staples, a prosthesis.

**14.** A method for gluing at least one hydrogel to at least one other article, wherein said method comprises:

a- applying a composition of nanoparticles on at least one face of the hydrogel
b- applying the face of the hydrogel bearing the nanoparticles to the article,

and wherein in case the at least one hydrogel or the at least one other article is a biological tissue, it is an isolated tissue or a cultured tissue.

**15.** A method according to claim 13, wherein step b is achieved underwater.

1a

1b

Figure 1

Figure 2a

Figure 2b

Figure 3a

Figure 3b

Figure 3c

Figure 4

Figure 5a

Figure 5b

Figure 5c

Figure 5d

Figure 6

Figure 7a

Figure 7b

Figure 8

Figure 9a

Figure 9b

Figure 9c

Figure 10

Figure 11

Figure 12

Figure 13

Figure 14

Figure 15

Europäisches
Patentamt

European
Patent Office

Office européen
des brevets

# EUROPEAN SEARCH REPORT

Application Number

EP 13 30 6243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AGUZZI ET AL: "Use of clays as drug delivery systems: Possibilities and limitations", APPLIED CLAY SCIENCE, ELSEVIER SCIENCE, NL, vol. 36, no. 1-3, 16 March 2007 (2007-03-16), pages 22-36, XP005932574, ISSN: 0169-1317, DOI: 10.1016/J.CLAY.2006.06.015 * page 29, right-hand column, last paragraph - page 30, right-hand column, paragraph first * ----- | 1-15 | INV. B82Y30/00 |
| | | | TECHNICAL FIELDS SEARCHED (IPC) |
| | | | B82Y A61K |

The present search report has been drawn up for all claims

| Place of search | Date of completion of the search | Examiner |
|---|---|---|
| Munich | 9 January 2014 | Vázquez Lantes, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................
& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120220911 A

**Non-patent literature cited in the description**

- **KENDALL, K.** Molecular Adhesion and Its Applications. Plenum Publishing Corporation, 2001
- **LAKE, G. J. ; THOMAS, A. G.** *Proceedings of the Royal Society A: Mathematical, Physical and Engineering Sciences,* 1967, vol. 300, 108-119
- **DE GENNES, P. G.** *Langmuir,* 1996, vol. 12, 4497-4500
- **SAHLIN, J. J. ; PEPPAS, N. A.** *Journal of Biomaterials Science, Polymer Edition,* 1997, vol. 8, 421-436
- **TAMAGAWA, H. ; TAKAHASHI, Y.** *Materials Chemistry and Physics,* 2008, vol. 107, 164-170
- **SAITO, J. et al.** *Polymer Chemistry,* 2011, vol. 2, 575
- **TECHAWANITCHAI, P. et al.** *Soft Matter,* 2012, vol. 8, 2844
- **GONG, J. P.** *Soft Matter,* 2006, vol. 2, 544
- **PEZRON, E. ; RICARD, A. ; LEIBLER, L.** *J. Polym. Sci. B Polym. Phys.,* 1990, vol. 28, 2445-2461
- **BOSMAN, A. W. ; SIJBESMA, R. P. ; MEIJER, E. W.** *Materials Today,* 2004, 34-39
- **REUTENAUER, P. ; BUHLER, E. ; BOUL, P. J. ; CANDAU, S. J. ; LEHN, J.-M.** *Chemistry,* 2009, vol. 15, 1893-1900
- **NICOLAÿ, R. ; KAMADA, J. ; VAN WASSEN, A. ; MATYJASZEWSKI, K.** *Macromolecules,* 2010, vol. 43, 4355-4361
- **IMATO, K. et al.** *Angew. Chem. Int. Ed.,* 2011, vol. 51, 1138-1142
- **CORDIER, P. ; TOUMILHAC, F. ; SOULIÉ-ZIAKOVIC, C. ; LEIBLER, L.** *Nature,* 2008, vol. 451, 977-980
- **MAES, F. et al.** *Soft Matter,* 2012, vol. 8, 1681-1687
- **BAÏT N. et al.** *Soft Matter,* 2011, vol. 7, 2025-2032
- **WANG, Q. et al.** *Nature,* 2010, vol. 463, 339-343
- **HARAGUCHI, K. ; UYAMA, K. ; TANIMOTO, H.** *Macromol. Rapid Commun.,* 2011, vol. 32, 1253-1258

- **CARLSSON, L. ; ROSE, S. ; HOURDET, D. ; MARCELLAN, A.** *Soft Matter,* 2010, vol. 6, 3619-3631
- **GAHARWAR, A. K. ; RIVERA, C. P. ; WU, C.-J. ; SCHMIDT, G.** *Acta Biomaterialia,* 2011, vol. 7, 4139-4148
- **DUARTE A.P. et al.** *Progress in Polymer Science,* 2012, vol. 37, 1031-1050
- **JOHNSEN, B. B. ; A. J. KINLOCH et al.** Toughening mechanisms of nanoparticle-modified epoxy polymers. *Polymer,* 2007, vol. 48 (2), 530-541
- **KINLOCH, A. J.** Adhesion and Adhesives: Science and Technology. Springer, 1987
- **ORTS-GIL, G. ; K. NATTE et al.** *Journal of Nanoparticle Research,* 2011, vol. 13 (4), 1593-1604
- **ALEXANDRIDIS, P. ; B. LINDMAN.** Amphiphilic Block Copolymers: Self-Assembly and Applications. Elsevier Science, 2000
- **HUNTER, R. J. ; L. R. WHITE.** Foundations of colloid science. Clarendon Press, 1987
- **HOURDET, D. ; L. PETIT.** Macromolecular Symposia. C. S. Patrickios. Wiley-V C H Verlag Gmbh, 2010, vol. 291-292, 144-158
- **WISNIEWSKA, M.** *Journal of Thermal Analysis and Calorimetry,* 2010, vol. 101 (2), 753-760
- Controlled growth of monodisperse silica spheres in the micron size range. *Journal of colloid and interface science,* 1968
- **VIA STÖBER et al.** Controlled growth of monodisperse silica spheres in the micron size range. *Journal of colloid and interface science,* 1968
- **PREVOTEAU, A. ; SOULIÉ-ZIAKOVIC, C. ; LEIBLER, L.** Universally Dispersible Carbon Nanotubes. *J. Am. Chem. Soc.,* 2012, vol. 134, 19961-19964
- **CARLSSON, L. ; ROSE, S. ; HOURDET, D. ; MARCELLAN, A.** Nano-hybrid self-crosslinked PDMA/silica hydrogels. *Soft Matter,* 2010, vol. 6, 3619-3631
- **KENDALL, K.** Cracking of Short Lap Joints. *The Journal of Adhesion,* 1975, vol. 7, 137-140

- **RIVLIN, R.S.** Large Elastic Deformations of Isotropic Materials .4. Further Developments of the General Theory. *Philosophical Transactions of the Royal Society of London Series a-Mathematical and Physical Sciences,* 1948, vol. 241 (835), 379-397
- **GREENSMITH, H.W.** Rupture of rubber. X The change in stored energy on making a small cut in a test piece held in simple extension. *Journal of Applied Polymer Science,* 1963, vol. 7 (3), 993-1002
- **HOURDET, D. ; L. PETIT.** Macromolecular Symposia. C. S. Patrickios. Wiley-V C H Verlag Gmbh, 2010, vol. 291-292, 144-158
- **PEFFERKORN, E. ; A. CARROY et al.** *Macromolecules,* 1985, vol. 18 (11), 2252-2258
- **WISNIEWSKA, M.** Temperature effect on adsorption properties of silica-polyacrylic acid interface. *Journal of Thermal Analysis and Calorimetry,* 2010, vol. 101 (2), 753-760
- **PARIDA SK ; DASH S ; PATEL S ; MISHRA BK.** *Advances in Colloid and Interface Science,* 2006, vol. 121 (1-3), 77-110
- **SEKINE Y ; IKEDA-FUKAZAWA T.** *Journal of Chemical Physics,* 2009, vol. 130 (3
- **HOURDET, D. ; PETIT, L.** *Macromol. Symp.,* 2010, vol. 291-292, 144-158

- **GRIOT, O. ; KITCHENER, J. A.** Role of surface silanol groups in the flocculation of silica suspensions by polyacrylamide. Part 1. Chemistry of the adsorption process. *Trans. Faraday Soc.,* 1965, vol. 61, 1026
- **HOURDET, D. ; PETIT, L.** Hybrid Hydrogels: Macromolecular Assemblies through Inorganic Cross-Linkers. *Macromol. Symp.,* 2010, vol. 291-292, 144-158
- **KENDALL, K.** Cracking of Short Lap Joints. *The Journal of Adhesion,* 1975, vol. 7, 137-140
- **JOHNER, A. ; JOANNY, J.-F.** Adsorption of polymeric brushes: Bridging. *J. Chem.Phys.,* 1992, vol. 96, 6257
- **SPRAKEL, J. ; VAN DER GUCHT, J. ; COHEN STUART, M. A. ; BESSELING, N. A. M.** Rouse, dynamics of colloids bound to polymer networks. *Phys. Rev. Lett.,* 2007, vol. 99, 208301
- **MONTARNAL, D. ; CAPELOT, M. ; TOURNILHAC, F. ; LEIBLER, L.** Silica-Like Malleable Materials from Permanent Organic Networks. *Science,* 2011, vol. 334, 965-968
- **GENT, A. N. ; HAMED, G. R. ; HUNG, W. J.** Adhesion of elastomer layers to an interposed layer of filler particles. *The Journal of Adhesion,* 2003, vol. 79, 905-913